# EUROPEAN PATENT APPLICATION

(11) **EP 4 403 112 A1**
(43) Date of publication of application: **24.07.2024**
(21) Application number: 24151523.8
(22) Date of filing: 12.01.2024
(51) Int. Cl.: A61B 6/03, A61B 6/00, A61B 6/02, A61B 6/51

(54) **APPARATUS AND METHOD FOR ACQUIRING DUAL ENERGY CBCT VOLUMETRIC RADIOGRAPHIES**

(30) Priority: 17.01.2023 IT 202300000525
(71) Applicant: CEFLA SOCIETA' COOPERATIVA, 40026 Imola (BO) (IT)
(72) Inventor: CHIAMPAN, Simone, 37057 San Giovanni Lupatoto (VR) (IT)
(74) Representative: Del Nero, Susanna

(57) **Abstract**

CBCT apparatus (1, 11) configured for acquiring patient's two distinct pluralities of X-ray raw projections to be combined for dual energy imaging, comprising an X-ray source and an X-ray detector, said X-ray source and detector being supported in an opposed position, the object under investigation being positioned in an intermediate position between said X-ray source and detector and in the propagation bundle of the radiation emitted by said X-ray source, moreover said X-ray source and detector being movable along a pre-set trajectory thanks to at least a rotational movement of said X-ray source and detector around a rotation axis (R),
wherein the acquisition of the first plurality of raw radiographic projections occurs with a first voltage of said X-ray source, while the acquisition of the second plurality of raw radiographic projections occurs with a second voltage of said X-ray source, different from the first voltage,
characterized in that
for acquiring the first plurality of raw projections at the first voltage, the rotation of said X-ray source and detector around the common axis of rotation (R) occurs in a direction opposite to the rotation direction of said X-ray source and detector around the common rotation axis (R) during the acquisition of the second plurality of raw radiographic projections at the second voltage, and in that
the patient maintains the same position during the acquisition of the second plurality of raw images, which occurs immediately after the acquisition of the first plurality of raw projections.

## Description

The present invention relates to a radiographic apparatus and method allowing to acquire volumetric 3D images. In particular, the invention relates to a particular mode of acquisition of said volumetric images, wherein an X-ray source set on two different voltages is used, known in the art as dual energy acquisition (see further).

In particular, the present invention finds advantageous, but not exclusive, application in cone-beam computerized tomography scanners used in dentistry, to which the ensuing description will make explicit reference, without for this implying any loss of generality.

In the present description the words detector or sensor are equivalent, meaning a unit receiving the radiation transmitted through the patient, and transforming the intensity of said radiation into electric signals corresponding to the intensity of said radiation.

Computerized tomography scanners provided with a passing gantry hosting a lying patient, used in dental practice and hospitals, are of the type comprising a table supporting a lying patient, and an X-ray source-detector assembly. Said X-ray assembly is designed to rotate around an area of analysis for acquiring raw projections of a particular anatomical area of interest of the patient, e.g. head, limbs, or portions of the vertebral column.

Typically, the patient's table is provided with a gurney which can slide inside and outside from the gantry, so as to acquire different anatomical areas of a patient. Typically, the patient takes her/his position on the table gurney while the gurney is in its extracted position with respect to the gantry. After this, the gurney is raised/lowered or laterally displaced so as to bring it in correspondence to the gantry hole, and then the gurney is slid inside the gantry up to a position suitable for acquiring the specific radiographic image; then the acquisition of raw projections can start. The acquisition takes place while the X-ray source and detector rotate around the patient. Once the acquisition is completed, the gurney is extracted from the gantry and the patient can stand up for releasing.

In dentistry, there are known cone-beam computerized tomography scanners acquiring images of a standing patient, too. Often, such apparatuses can acquire different kinds of images, e.g. computerized tomographic images and even panoramic images of the dental arches of a patient. In this case, too, the apparatus comprises an X-ray source and detector fixed to the ends of a rigid support, while the patient is positioned in an intermediate position between said X-ray source and detector. In the known art, apparatuses are known wherein the X-ray source and detector are moved according to pre-set trajectories thanks to the combination of three movements, i.e. a rotational movement around an axis of rotation of the rigid support, through which X-ray source and detector are moved along a circular trajectory around said axis and around the patient, and a translational movement of the axis of rotation of support arm according to at least one or two different directions in the horizontal plane perpendicular to the axis of rotation of the support arm.

Both in the case of an apparatus for lying patients, and in the case of an apparatus for standing patients, the radiographic technique is cone-beam volumetric radiography (also known as CBCT or 3D), i.e. the acquisition from different projection angles, of a series of two-dimensional raw radiographic projections, which will be processed post-acquisition to reconstruct three-dimensional volumes.

The apparatus further comprises a control unit, connected to the source-detector assembly, for controlling emission and reception of the X-ray beam in a way synchronous with the rotation of the arm, and a processing unit connected to the detector for receiving, storing, and processing the raw tomographic projections so as to reconstruct images of the object. The processing unit is provided with a screen in order to allow the visualization of the reconstructed images.

In the art, it is known that the value of the energy emitted by the X-ray source (i.e. the voltage of the X-ray tube, kVp) affects the diagnostic quality of radiographic images.

In particular, when soft tissues are acquired, the differences in CT value (hounsfield units) are large at relatively low energy, providing clear contrast and making it easy to diagnostically image the soft tissue, but the differences in CT value are small at relatively high energy, providing low contrast and making it difficult to diagnostically image the soft tissue.

On the other hand, high energy is advantageous for acquiring hard tissues, for the crossing of the radiation on wide anatomical districts with consequent noise reduction, and for artefact reduction in reconstructed volumes, due to X-ray beam hardening issues.

Indicatively, for high energy (high voltage) of the X-ray tube values around 100-130 kVp are meant, while for low energy (low voltage) values around 60-90 kVp are meant.

In the art it is known applying a radiographic technique wherein the same anatomical area is acquired using two different voltages of the X-ray source, which raw projections are then combined and processed in order to obtain a final dual energy image allowing a more accurate diagnosis. Said radiographic technique is known as dual energy scanning or multi-energy scanning.

In the art, there are known at least four dual energy or multi-energy modes:
1. Slow-kV switching mode
   This first method provides a double rotation of the radiographic system around the patient, wherein a first plurality of raw radiographic projections is taken at a first X-ray tube voltage and a second plurality of raw projections is taken at a second X-ray tube voltage, different from the first X-ray tube voltage, using a single X-ray tube.
   As the raw projections of the two acquisition must be combined in order to get the final reconstructed image, in this method the immobility of the patient is critical: if the patient moves, the two acquisitions cannot be combined to obtain a final reconstructed image with a better diagnostic value. In fact, the acquisition time of the radiographic projections is at least double with respect to the acquisition of raw projections with a standard mode (with just one voltage of the X-ray tube), which increases the probabilities that the patient moves during the acquisition of the first and second raw projections.
2. Fast-kV switching mode or high-speed switching mode
   This second method provides a single rotation of the radiographic system around the patient, wherein a rapid switching of the X-ray tube voltage occurs on a view by view basis during rotation (scanning). E.g., at the degree 1 of the rotation of the radiographic system a raw projection is acquired with the first voltage, at the degree 2 of the rotation a raw projection is acquired with the second voltage, at the degree 3 of the rotation a raw projection is acquired with the first voltage, at the degree 4 of the rotation a raw projection is acquired with the second voltage, etc., covering all the degrees of the acquisition trajectory. Alternatively, in a plurality of raw projections, all the odds projections are acquired with the first voltage and the even projections are acquired with the second voltage of the X-ray tube, different from the first.
   In this mode, the X-ray detector will read data twice, high energy projection data the first time and low energy projection data the second time. The voltage switching of the X-ray tube must be very fast, and in this case, too, the cost of the apparatus is very high, while the control of the tube voltage is very complex.
3. Dual source system
   This third method provides taking projections at different X-ray tube voltages using two X-ray tubes set at different voltages, rather than a single X-ray tube. A single acquisition may be provided, wherein both X-ray tubes acquire raw projections at the same time.
   This method is very expensive, too, because it requires providing two X-ray tubes in the same radiographic apparatus.
4. Multilayer system
   This fourth method X-ray provides the use of detectors of multilayered structure, e.g. detector with a shallow layer and detector with a deep layer. For example, when X-ray detectors of a two-layer structure are used, low-energy X-rays are detected by the detector in the shallow layer and high-energy X-rays passing through the shallow layer are detected by the detector in the deep layer.
   This method is expensive, too, because it requires the use of sophisticated sensors; the method does not ensure a suitable energetic separation of the filtered X-ray beam. Moreover, with this technique, the apparatus administers a high X-ray dose even when non-dual energy images are acquired.

In the art, dual energy radiographic images mean a set of types of reconstructed volumetric images that are obtained by combining the raw projections acquired at different energies. There are many combination methods, and they allow to obtain various types of images, each useful for specific diagnostic purposes. E.g., with this technique the following dual energy images can be obtained, that are known in the art:
- VMI (Virtual Monoenergetic Images) volumetric images, i.e. images that have the same characteristics as images theoretically reconstructed with a mono-energetic beam and which, as such, are free from beam-hardening and have fewer effects from metal artefacts;
- Blended volumetric images, i.e. weighted combination, even nonlinear, of the images obtained in correspondence with the two energies in order to obtain images that exploit the greater contrast of the low energy and the lower noise of the high energy;

- Volumetric images with suppression of one component (e.g. bone) to better visualize other tissues (e.g. VNCa=Virtual NoCalcium);
- Volumetric images with contrast agent subtraction (VNC=Virtual Non-Contrast images) which allow to avoid exposing the patient to the blank scan before the contrast medium enters into the patient;
- Volumetric images with tissue characterization, i.e. identification of the tissue composition of a body area, with the possibility of discriminating up to three components of a mixture and consequent production of so-called colour coded images, with associated colors linked to the concentration of the various components/material.

US20170135651A1 describes a dual energy acquisition apparatus and method. The apparatus is configured to carry out a first scan along a first helix-segment-shaped trajectory section and to carry out a second scan along a second helix-segment shaped trajectory section. A first data set is obtained during the first scan, and a second data set is obtained during the second scan. Taken by themselves in each case, both the first data set and the second data set are too incomplete for a reconstruction of a volume image without a partial revolution artefact. From the two data sets, a fused three- or four-dimensional data set is generated that is sufficiently complete for a reconstruction of a volume image without a partial revolution artefact. Two pairs of X-ray source and detector are provided, and the apparatus is provided with a separate C-arm for each pair of X-ray source and detector.

KR20170045175A describes a dual energy acquisition apparatus and method, and more particularly, a cone-beam tomography technique which can acquire a medical image as well as a luggage test. Said apparatus includes a first gantry in which a first X-ray source and a first detector are installed, and a second gantry in which a second X-ray source and detector are installed, and a processor for controlling the imaging of the object while rotating the first gantry and the second gantry, wherein the processor emits different energies to the first X-ray source and the second X-ray source. The first gantry and the second gantry are rotated in a first direction from a first angle to a second angle, and the first gantry and the second gantry are rotated in a second direction from a second angle to a first angle.

US20200170591A1 describes a dual energy acquisition apparatus and method. The apparatus is provided with just one X-ray source and detector and receives first projection data measured by the detector during the first pass, and second projection data measured by the detector during the second pass; a patient image is reconstructed based on the first projection data and the second projection data.

Aim of the present invention is providing a radiographic apparatus allowing to use the dual energy mode of acquisition, obtaining dual energy diagnostic images provided with a better quality, without the drawbacks of the prior art.

This object is achieved by an apparatus and a method having the features of the independent claims. Advantageous embodiment and refinements are specified in the claims dependent thereon.

The present invention provides acquiring diagnostic images in dual energy mode, in particular according to the Slow-kV switching mode, wherein the first acquisition of raw projections with the first voltage of the X-ray tube is performed in a direction (e.g. clockwise), while the second acquisition of raw projections is performed in the opposed direction (e.g. anti-clockwise). The first and the second acquisition are performed one after the other, without moving the patient. In this way, acquisition times are shortened, preventing the occurrence of motion artefacts.

In a first embodiment, there is provided that the acquisition of the raw projections occurs on a trajectory of 360°, travelling first in a direction and then in the opposed direction.

In a second, preferred embodiment, there is provided that the acquisition of the raw projections occurs on a trajectory shorter than 360°, indicatively 200°, travelling first in a direction and then in the opposed direction. In this way, there is a further shortening of acquisition times.

In both the 360° embodiment and in the shorter than 360° embodiment, the raw projections are preferably acquired at the same angles.

In an embodiment, between the first and the second acquisition of raw projections, there is a change through repositioning of the output filter for the X-ray beam crossing the patient.

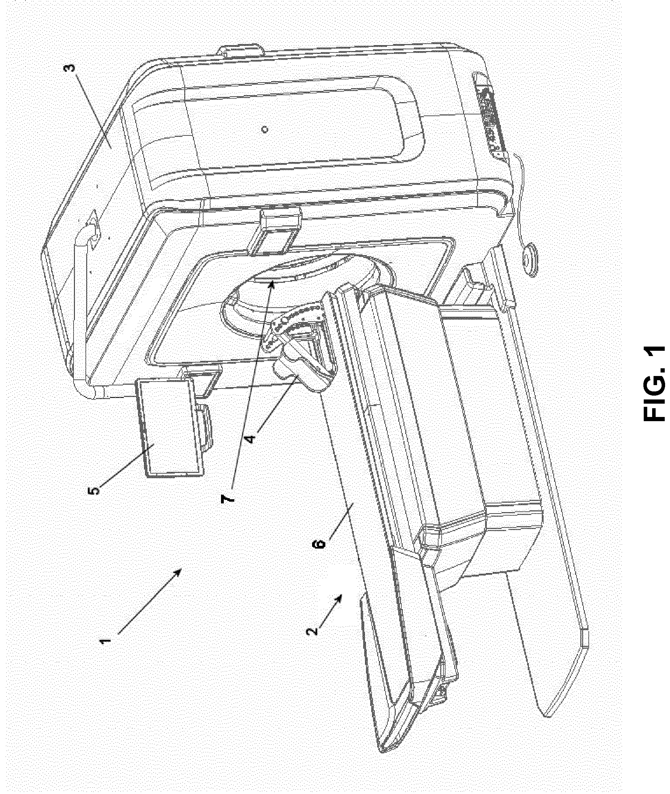

In an embodiment, the dual energy mode is applied on a radiographic apparatus wherein the patient is lying (see Figure 1).

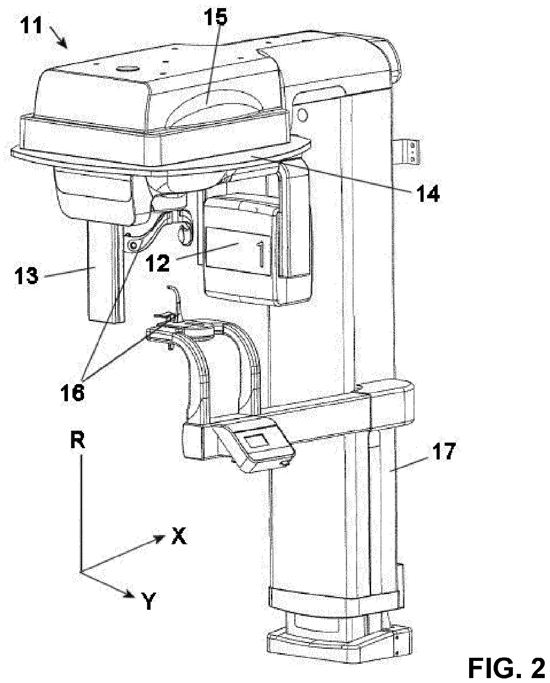

In an embodiment, the dual energy mode is applied on a radiographic apparatus wherein the patient is standing (see Figure 2).

The method for the acquisition of a dual energy reconstructed image according to the present invention comprises the following steps:
- Positioning of the lying or standing patient inside the apparatus, with a first rough patient centring based on a laser pointing system;
- Selecting the desired acquisition protocol by a human operator;
- Acquiring at least two scout views at different angles, giving indication to the human operator on the proper positioning of the patient;
- Optionally positioning the X-ray source filter specific for the first acquisition, preferably the high-energy acquisition;
- Once the patient is properly positioned, acquisition of the first raw projections, preferably the high-energy projections; the system X-ray source-detector rotates in a first direction, preferably clockwise;
- Optionally positioning the X-ray source filter specific for the second acquisition, preferably the low-energy acquisition;
- Acquisition of the second raw projections, preferably the low-energy projections; the system X-ray source-detector rotates in a second direction, opposed to the first direction, preferably anticlockwise;
- Processing of the raw projections in order to obtain a final reconstructed dual energy image, or alternatively two distinct images, one at high and one at low energy.

A first advantage of the present invention is the possibility of performing dual energy CBCT acquisitions, improving the diagnostic quality of the radiographic images and reducing the occurrence of motion artefacts.

A second advantage is performing dual energy CBCT acquisitions with the cheapest of the four known methods, obtaining reconstructed dual energy images of a better diagnostic quality, capable of representing at their best both hard tissues and soft tissues.

A third advantage of the present acquisition is that, in the preferred embodiment of the acquisition trajectory shorter than 360°, the present invention provides a further shortening of acquisition times, still using a dual energy acquisition technique.

A fourth advantage consists in the fact that the first high energy acquisition and the second low energy raw acquisition can be combined in a final reconstructed dual energy image, or can be processed in order to obtain two separate images, a first image wherein hard tissues are well imaged, and a second image wherein soft tissues are well imaged.

A fifth advantage is, as raw projections are acquired in opposed directions but at the same angles, in case of patient's movement between the first and the second acquisition, the patient's movement can be compensated through software techniques so as to reduce motion artefacts.

It is worth noting that keeping the patient under radiographic acquisition in a tranquil state wherein she/he can maintain the immobility required for the whole duration of the acquisition is very important. As a matter of fact, both the reduced dimensions of the gantry hole or of the positioning system for the patient, and the noises emitted by the X-ray source-detector system during the acquisition may induce stress in patients, up to triggering claustrophobia attacks in more phobic patients.

Further advantages and properties of the present invention are disclosed in the following description, in which exemplary embodiments of the present invention are explained in detail based on the drawings:
- Figure 1: Axonometric view of a radiographic apparatus for a lying patient according to the present invention;
- Figure 2: Axonometric view of a radiographic apparatus for a standing patient according to the present invention;
- Figure 3: First embodiment, acquisition of raw projections on a trajectory of 360°, schematic view;
- Figure 4: Second embodiment, acquisition of raw projections on a trajectory shorter than 360°, schematic view.

Figure 1 shows an extraoral radiographic apparatus 1 capable of producing CBCT images, comprising a table 2 provided with a gurney 6 for positioning a (not shown) patient in a lying position, and a gantry 3, in its turn comprising a not shown X-ray source and detector.

Said patient table 2 optionally comprises an adjustable device 4 for immobilizing patient's head. Said gantry 3 comprises a screen 5 to visualize the patient's images or an instruction panel. Said gantry 3 further comprises a hole 7 allowing the entrance of the table gurney 6 which supports the patient.

In a known way, said gantry hides from view the X-ray source-detector group; in a known way, the X-ray source-detector group rotates around the (not shown) patient during the acquisition of raw projections.

Figure 2 shows a radiographic apparatus 11, wherein projections are acquired while the (not shown) patient is standing; typically, said apparatus 11 can acquire both tomographic images with cone beam technique (CBCT) and panoramic images. Said extraoral apparatus 11 comprises an X-ray source 12 projecting a collimated X-ray bundle across a (not shown) patient, a bi-dimensional X-ray detector 13 positioned so as to measure the intensity of radiation after it crossed the patient, a C-arm 14 on which said X-ray source 12 and detector 13 are fixed, a mechanical system 15 rotating and translating said support C-arm 14 around the patient so that said C-arm moves said X-ray source and detector around the patient, so as to acquire radiographic projections from different positions.

The mechanical system 15 is provided with at least one, possibly three, different degrees of freedom of movement, which allow the rotation of C-arm 14 around an axis R perpendicular to the longitudinal extension of the arm, and intermediate between X-ray source 12 and detector 13, and the translation of said axis of rotation R according to at least one, possibly two, different directions X, Y in a plane perpendicular to said axis of rotation R of C-arm 14. The radiographic apparatus 11 further comprises a (not shown) electronic system to control and synchronize the working of the various apparatus components. Said translation of the axis of rotation R is provided for acquiring panoramic images.

Moreover, the apparatus 11 comprises a device 16 for positioning the patient. In the non-limiting example of Figure 2, the patient positioning device 16 consists of a bite and supports for positioning patient's skull. The position of C-arm 14 can be adjusted to patient's height thanks to a vertically mobile post 17.

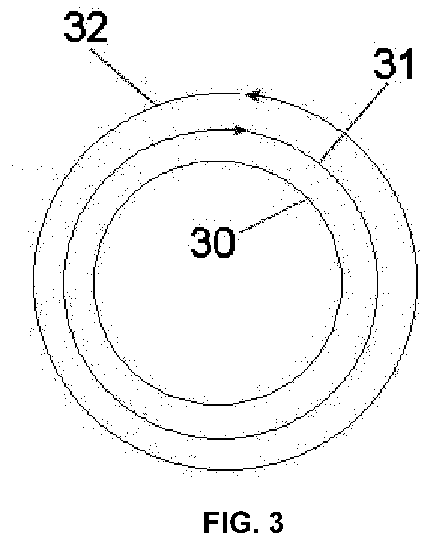

Figure 3 shows the first embodiment according to the present invention, wherein during the acquisition of raw projections the X-ray source-detector group rotates around the patient, schematically represented with the numeral 30, for 360°. During the first acquisition of raw projections, the X-ray source-detector group rotates in a first direction 31 (e.g. clockwise), while during the second acquisition the same group rotates in the opposed direction 32 (e.g. anti-clockwise). During the first acquisition of raw projections the X-ray tube takes raw projections with a first voltage, while during the second acquisition the X-ray tube takes raw projections with a second voltage, different from the first voltage.

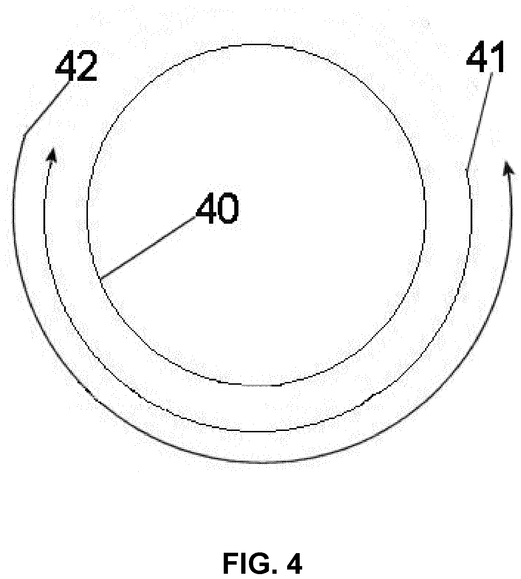

Figure 4 shows the second embodiment according to the present invention, wherein during the acquisition of raw projections the X-ray source-detector group rotates around the patient for a trajectory shorter than 360°, e.g. 200°. During the first acquisition of raw projections, the X-ray source-detector group rotates around the patient, schematically represented with the numeral 40, in a first direction 41 (e.g. clockwise), while during the second acquisition the same group rotates in the opposed direction 42 (e.g. anticlockwise). During the first acquisition of raw projections the X-ray tube takes raw projections with a first voltage, while during the second acquisition the X-ray tube takes raw projections with a second voltage, different from the first voltage.

In both cases, the plurality of raw projections acquired during the first acquisition and the second acquisition is combined and processed to obtain a dual energy final image provided with a better diagnostic quality in comparison to what can be obtained using just one voltage. In case of patient's movements between the first and the second acquisition, the motion can be compensated using known software techniques, so reducing/removing the motion artefact.

In an embodiment, there is a change through repositioning of the output filter for the X-ray beam between the first and the second acquisition, in order to optimize the average energy of the X-ray beam to increase the effective energy difference between the two acquisitions. The apparatus is provided with two (not shown) filters placed on a (not shown) alternating device allowing to place in front of the X-ray source the desired filter, for the high or low energy acquisition. Said alternating devices and filters specific for the X-ray tube voltage are known by the skilled man.

The X-ray source is an X-ray tube controlled by a high voltage generator, in its turn controlled by a control electronics in order to obtain the two voltages needed for the first and the second acquisition.

The mechanism of inversion of the acquisition trajectory from the clockwise direction into the anticlockwise direction exploits the device already present in non-dual energy apparatuses for resetting the apparatus, therefore the apparatus does not need to be modified in its mechanics.

In practice, the acquisition of a dual energy reconstructed image comprises the following steps:
- Positioning of the lying or standing patient inside the apparatus 1 or 11, with a first rough patient centring based on a laser pointing system; laser pointing systems are known in the art;
- Selecting the desired acquisition protocol by a human operator;
- Acquiring at least two scout views at different angles, giving indication to the human operator on the proper positioning of the patient, the acquisition of scout views is known in the art;
- Optionally positioning the X-ray source filter specific for the first acquisition, preferably the high-energy acquisition;
- Once the patient is properly positioned, acquisition of the first raw projections, preferably the high-energy projections; the system X-ray source-detector rotates in a first direction, preferably clockwise;
- Optionally positioning the X-ray source filter specific for the second acquisition, preferably the low-energy acquisition;
- Acquisition of the second raw projections, preferably the low energy projections; the system X-ray source-detector rotates in a second direction, opposed to the first direction, preferably anticlockwise;
- Processing of the raw projections in order to obtain a final reconstructed dual energy image, or alternatively two distinct images, one at high and one at low energy.
Should the patient have moved, the software processing the raw projections provides to compensate for the motion artefact.

It is well noting that, according to the present invention, the dual energy mode is supplementary to the radiographic acquisitions that can be performed with the CBCT apparatuses presently on the market, in that it requires a minimal mechanical modification (providing a specific filter for the X-ray bundle), and an updating of the software for acquiring and processing images. This improves the diagnostic quality of the CBCT apparatus; optionally the retrofitting of CBCT apparatuses already installed is possible.
- 1: extraoral radiographic apparatus with lying patient
- 2: table
- 3: gantry
- 4: device for immobilizing patient's head
- 5: screen
- 6: gurney
- 7: gantry hole
- 11: extraoral radiographic apparatus with standing patient
- 12: X-ray source
- 13: X-ray detector
- 14: C-arm
- 15: mechanical system
- 16: devices for positioning patients
- 17: post
- 30: patient
- 31: trajectory of the first acquisition
- 32: trajectory of the second acquisition
- 40: patient
- 41: trajectory of the first acquisition
- 42: trajectory of the second acquisition

## Claims

1. CBCT apparatus (1, 11) configured for acquiring patient's two distinct pluralities of X-ray raw projections to be combined for dual energy imaging, comprising an X-ray source and an X-ray detector, said X-ray source and detector being supported in an opposed position, the object under investigation being positioned in an intermediate position between said X-ray source and detector and in the propagation bundle of the radiation emitted by said X-ray source, moreover said X-ray source and detector being movable along a pre-set trajectory thanks to at least a rotational movement of said X-ray source and detector around a rotation axis (R),
wherein the acquisition of the first plurality of raw radiographic projections occurs with a first voltage of said X-ray source, while the acquisition of the second plurality of raw radiographic projections occurs with a second voltage of said X-ray source, different from the first voltage,
**characterized in that**
for acquiring the first plurality of raw projections at the first voltage, the rotation of said X-ray source and detector around the common axis of rotation (R) occurs in a direction opposite to the rotation direction of said X-ray source and detector around the common rotation axis (R) during the acquisition of the second plurality of raw radiographic projections at the second voltage,
and **in that**
the patient maintains the same position during the acquisition of the second plurality of raw images, which occurs immediately after the acquisition of the first plurality of raw projections.

2. CBCT apparatus (1, 11) configured for acquiring patient's two distinct pluralities of X-ray raw projections to be combined according to claim 1, wherein the X-ray source and detector acquire the first and the second plurality of raw projections by rotating for a trajectory of 360° around said patient.

3. CBCT apparatus (1, 11) configured for acquiring patient's two distinct pluralities of X-ray raw projections to be combined according to claim 1, wherein the X-ray source and detector acquire the first and the second plurality of raw projections by rotating for a trajectory shorter than 360° around said patient.

4. CBCT apparatus (1, 11) configured for acquiring patient's two distinct pluralities of X-ray raw projections to be combined according to one or more of the preceding claims, wherein the first high energy voltage of the X-ray tube ranges 100-130 kVp, while the second low energy voltage of the X-ray tube ranges 60-90 kVp.

5. CBCT apparatus (1, 11) configured for acquiring patient's two distinct pluralities of X-ray raw projections to be combined according to one or more of the preceding claims, wherein there is provided a first X-ray beam filter for the X-ray beam impinging on the patient, that is specific for the first acquisition and a second X-ray beam filter for the X-ray impinging on the patient, that is specific for the second acquisition, said second filter replacing the first filter between the first and the second acquisition.

6. CBCT apparatus (1, 11) configured for acquiring patient's two distinct pluralities of X-ray raw projections to be combined according to one or more of the preceding claims, wherein the acquisition of the raw projections occurs at the same angles in the first and in the second acquisition.

7. CBCT apparatus (1, 11) configured for acquiring patient's two distinct pluralities of X-ray raw projections to be combined according to claim 6, comprising a software for image processing suitable for compensating for the artefact due to patient's motion between the first and second acquisition.

8. CBCT apparatus (1) configured for acquiring patient's two distinct pluralities of X-ray raw projections to be combined according to one or more of claims 1-7, comprising a table (2) on which a patient is lying for acquiring said raw projections, and a gantry (3) comprising the X-ray source-detector group, said table being provided with a gurney (6) sliding inside and outside said gantry, so as to acquire different anatomical areas of said patient.

9. CBCT apparatus (11) configured for acquiring patient's two distinct pluralities of X-ray raw projections to be combined according to one or more of the claims 1-7, comprising an X-ray source (12) projecting a collimated X-ray bundle across a standing patient, a bi-dimensional X-ray detector (13) positioned so as to measure the intensity of radiation after it crossed the patient, a C-arm (14) on which said X-ray source (12) and detector (13) are fixed, said C-arm (14) being provided with at least one movement, the first being a rotational movement around the axis of rotation around (R), and optionally with at least a second translational movement of the axis of rotation (R) along a direction (Y) in the horizontal plane perpendicular to said axis of rotation (R) or with another rotational movement of the rotation axis (R) of C-arm (4) around a second axis of rotation; the position of C-arm (14) being adjusted to patient's height thanks to vertically mobile post (17); a mechanical system (15) so as to allow rotation and translation of said C-arm around the patient, so as to acquire raw projections from different positions; a device (16) for positioning said patient; an electronic system controlling and synchronizing the working of the various components of the apparatus.

10. Method for acquiring patient's two distinct pluralities of X-ray raw projections to be combined for dual energy imaging, said method being executed through a CBCT apparatus (1, 11) according to one or more of claims 1-9, comprising the following steps:
- Positioning of the lying or standing patient inside the apparatus (1 or 11), with a first rough patient centring based on a laser pointing system;
- Selecting the desired acquisition protocol by a human operator;
- Acquiring at least two scout views at different angles, giving indication to the human operator on the proper positioning of the patient;
- Optionally positioning the X-ray source filter specific for the first acquisition;
- Once the patient is properly positioned, acquiring the first raw projections with a first voltage; the system X-ray source-detector rotates in a first direction;
- Optionally positioning the X-ray source filter specific for the second acquisition;
- Acquiring the second raw projections with a second voltage, different from the first voltage; the system X-ray source-detector rotates in a second direction, opposed to the first direction;
- Processing the raw projections in order to obtain a final reconstructed dual energy image, or alternatively two distinct images, one at high and one at low energy.

11. Method according to claim 10 for acquiring patient's two distinct pluralities of X-ray raw projections to be combined through an apparatus (1, 11), said method comprising the following steps:
- Positioning of the lying or standing patient inside the apparatus (1 or 11), with a first rough patient centring based on a laser pointing system;
- Selecting the desired acquisition protocol by a human operator;
- Acquiring at least two scout views at different angles, giving indication to the human operator on the proper positioning of the patient;
- Optionally positioning the X-ray source filter specific for the first high-energy acquisition;
- Once the patient is properly positioned, acquiring the first raw high-energy projections; the system X-ray source-detector rotates in a first clockwise direction;
- Optionally positioning the X-ray source filter specific for the second low-energy acquisition;
- Acquiring the second raw low-energy projections; the system X-ray source-detector rotates in a second anticlockwise direction, opposed to the first direction;
- Processing the raw projections in order to obtain a final reconstructed dual energy image, or alternatively two distinct images, one at high and one at low energy.
